(19) Europäisches Patentamt
European Patent Office
Office européen des brevets

(11) Veröffentlichungsnummer: **0 514 651 A2**

## (12) EUROPÄISCHE PATENTANMELDUNG

(21) Anmeldenummer: **92105571.1**

(22) Anmeldetag: **01.04.92**

(51) Int. Cl.5: **A61B 17/36**

(30) Priorität: **15.04.91 DE 9104559 U**

(43) Veröffentlichungstag der Anmeldung:
**25.11.92 Patentblatt 92/48**

(84) Benannte Vertragsstaaten:
**DE ES FR GB IT**

(71) Anmelder: **SÖRING GmbH MEDIZINTECHNIK**
**Justus-von-Liebig-Ring 10**
**W-2085 Ouickborn(DE)**

(72) Erfinder: **Söring, Holger**
**Marienhöhe 72**
**W-2085 Ouickborn(DE)**

(54) **Mikro-Elektro-Schutzgas-Schweissgerät.**

(57) Die Erfindung betrifft ein Mikro-Elektro-Schutzgas-Schweißgerät, welches von einer Versorgungsstation über in einem Griffstück **1** untergebrachte Versorgunsleitungen **11,13** für Schutzgas und hochfrequente Spannung versorgt wird. An dem Griffstück ist der Elektrodenhalter und ein induktiv erwärmbares Messer angeordnet, außerdem sind Betätigungsschalter **23** für die Regulierung von Gasstrom und Spannung vorgesehen.

EP 0 514 651 A2

Die Erfindung bezieht sich auf ein Mikro-Elektro-Schutzgas-Schweißgerät mit in einem Griffteil untergebrachten Versorgungsleitungen für das Schutzgas und die hochfrequente Spannung, sowie einem Elektrodenhalter und einer die Elektrode umgebenden Düse, durch die das in einem auch die elektrischen Leiter aufnehmenden Schlauch von der Gasversorgungseinrichtung zugeführte Gas auf das zu behandelnde Objekt austritt.

Derartige Mikro-Elektro-Schutzgas-Schweißgeräte werden in verschiedenen Bereichen eingesetzt. Insbesondere dort, wo der Lichtbogenbereich -beim Verbinden von Metallen die Schmelzzonevom Sauerstoff abgeschlossen sein muß, um eine Oxidation der zu behandelnden Teile oder Oberflächen zu verhindern.

So sind auch Geräte im Einsatz zur Wärmebeaufschlagung, zur Eiweißdenaturierung, zur artifiziellen Hämatose, zur Nekrotisierung von Oberflächen etc.

Besonders im Medizinbereich in der Parenchymchirurgie werden derartige Geräte gern bei Leber- und Milzoperationen eingesetzt, um die Kapillargefäße zu verschweißen und so den Blutverlust des Patienten erheblich zu verringern. Hierbei ist es außerdem vorteilhaft, daß sich nicht abnutzende Elektroden eingesetzt werden können.

Der Einsatz und die Entwicklung derartiger Schutzgas-Schweißgeräte sind in verschiedenen Patentanmeldungen mehr oder weniger ausführlich beschrieben.

Sehr ausführlich wird in der DE OS 37 10 489 eine Vorrichtung zur elektrochirurgischen Koagulation und Desikkation beschrieben. So soll es die in dieser Patentanmeldung beschriebene Vorrichtung, bei der zur elektrochirurgischen Koagulation elektrische Energie in Lichtbögen zu einem von Blut durchströmten Gewebe in einem Lebewesen geleitet wird, ermöglichen, daß durch Leiten eines insgesamt laminaren Strahls ionisierbaren Gases Blut von der Oberfläche eines Gewebes entfernt werden, wobei durch das Leiten der eiektrischen Energie in Lichtbögen ein Schorf mit vorbestimmten Eigenschaften erzeugt werden soll.

Das hierfür erforderliche stiftartige Handstück, das durch den Chirurgen während der chirurgischen Prozedur manipulierbar ist, weist eine Düse zum Erzeugen des Gasstrahles und eine Einrichtung zum Übertragen der elektrischen Energie mittels einer Elektrode in dem Gasstrahl auf, wobei eine Schnur das Handstück mit einer Gasversorgungseinrichtung und einem Generator verbindet. Diese Schnur ist so gestaltet, daß über ihre Länge mehrere um einen elektrischen Leiter angeordnete Gasleithohlräume angeordnet sind,die sich insgesamt parallel zu dem elektrischen Leiter erstrecken.

Das stiftartige Handstück besteht aus einem Griff, der mit der Schnur verbunden ist, einer Düsen-und Elektrodenhalteeinrichtung, mit der die Düse und die Elektrode integral verbunden sind und einer Kupplungseinrichtung, die mit dem Griff verbunden ist zum lösbaren Verbinden der Düsen- und Elektrodenhalteeinrichtung mit dem Haltestück, wobei die Kupplungseinrichtung außerdem die Elektrode mit dem elektrischen Leiter der Schnur elektrisch verbindet und außerdem Gas aus dem Gasleitkanal in die Düse leitet.

Nachteilig an dieser sehr aufwendigen Konstruktion ist, daß aufgrund der zentrischen Anordnung des elektrischen Leiters in dem Gasführungsschlauch dieser elektrische Leiter im Bereich des Anschlusses an die Gasversorgungseinrichtung mit besonderem Aufwand abgedichtet werden muß. Außerdem ist eine Vielzahl von Kupplungsteilen notwendig, die alle sorgfältig abgedichtet werden müssen. Dieses führt nicht nur zu sehr hohen Fertigungskosten, sondern es können auch Undichtigkeiten auftreten, die eine besondere Gefahr für den Chirurgen darstellen.

Der vorliegenden Erfindung liegt die Aufgabe zugrunde, ein Mikro-Elektro-Schutzgas-Schweißgerät mit in einem Griffteil untergebrachten Versorgungsleitungen für das Schutzgas und die hochfrequente Spannung, sowie einem Elektrodenhalter und einer die Elektrode umgebenden Düse zu schaffen, weiches die beschriebenen Nachteile nicht aufweist und weiches außerdem einfach aufgebaut ist und preiswert hergestellt werden kann.

Die Lösung dieser Aufgabe besteht darin, daß ein Mikro-Elektro-Schutzgas-Schweißgerät sich dadurch auszeichnet, daß Elektrodenhalter und Gaszuführ- und verteilungskörper einstückig ausgeführt sind und der Elektrodenhalter elektrisch leitend mit einem Zentrierelement verbunden ist.

Weitere zweckdienliche Ausgestaltungen eines solchen Schweißgerätes sind in den Unteransprüchen erläutert.

Ein Ausführungsbeispiel der Erfindung ist in der folgenden Beschreibung im Zusammenhang mit den Zeichnungen beschrieben:
Es zeigt:

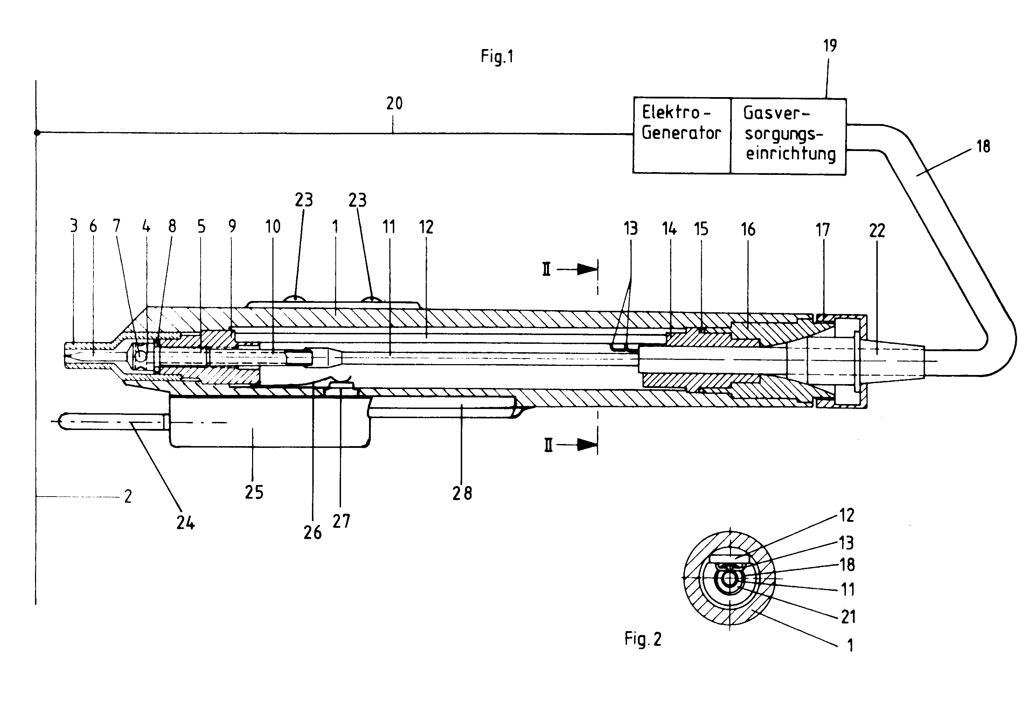

Fig. 1      einen Längsschnitt durch ein Schweißgerät,

Fig. 2      einen Querschnitt entspr. der Linie II - II aus Fig. 1

Das erfindungsgemäße Mikro-Elektro-Schutzgas-Schweißgerät besteht aus einem Griffstück **1**, welches eine Düse **3**, die Gaszuführeinrichtung und die elektrische Zuleitung aufnimmt.

Die Düse **3** besteht aus einem elektrisch nicht leitenden Material, vorzugsweise aus Keramik.Diese Düse **3** umschließt eine aus einem sich nicht abnutzenden Material bestehende Elektrode **6**. Vorzugsweise wird hierfür Wolfram als Material gewählt. Diese Elektrode **6** ist in einem Elektrodenhalter **4** befestigt. Der Elektrodenhalter **4** ist mittels eines Gewindes **5** in einem Zentrierelement **9** elektrisch leitend befestigt.

Das Gewinde **5** geht in eine Schlauchaufnahme **10** über, auf die der Gasschlauch **11** aufgeschoben ist. Im elektrodennahen Bereich des Elektrodenhalters **4** sind Gasaustrittsöffnungen **7** vorgesehen, aus denen das durch den Schlauch zugeführte Gas ausströmen kann. Damit Gas nicht in den Innenraum des Griffstückes **1** gelangen kann, ist zwischen Elektrodenhalter **4** und Zentrierelement **9** ein Dichtring **8** vorgesehen. Dieser Dichtring **8** ist so gestaltet, daß er den Eiektrodenhalter **4** gegen das Zentrierelement **9** abdichtet.

An dem Zentrierelement **9** ist die Leiterplatte **12** so befestigt, daß die hierauf vorgesehenen - nicht dargestellten- Leiterbahnen, die die Elektrode **6** mit hochfrequenter Spannung versorgen sollen, in elektrisch leitender Verbindung zum Zentrierelement **9** stehen.

Ferner können an dem Griffstück **1** Mikrotaster und - schalter **23** angeordnet sein, mit deren Hilfe der Benutzer die elektrische Verbindung herstellen und trennen kann. Außerdem ist es möglich, die Stromstärke zu steuern und den Gasfluß zu verstärken oder zu vermindern.

Die an der Leiterplatte **12** befestigten Steuerkabel **13** sind zusammen mit dem Gasschlauch in einem Schutzschlauch untergebracht. Die Zwischenräume zwischen Kabel **13** und Gasschlauch **11** sind mit einem hitsebeständigen Material vorsugsweise Silikon **21** ausgefüllt.

Dieser Schutzschlauch **18** stellt die Verbindung des Schweißgerätes mit der Versorgungseinheit **19** her. Diese Versorgungseinheit **19** ist mit allen für die Gas- und Spannungsregelung erforderlichen Einrichtungen ausgestattet.

Auf der den Tastern **23** gegenüber liegenden Seite ist eine Führung **28** für einen Schieber **25**, der bei einer Verlagerung in Achsrichtung des Griffstückes die leitende Verbindung zwischen einem Kontkatstift **27** und einer mit der Leiterplatte **12** verbundenen Kontaktfeder **26** herstellt. Durch diese elektrische Verbindung wird eine - hier nicht dargesteilte Induktionsspule - mit Spannung versorgt, so daß das in den Schieber **25** eingesetzte Messer **24** erhitzt wird. Mit diesem Messer kann der Chirurg Gewebe durchtrennen. In zurückgezogener Position ist das Messer nicht erwärmt, so daß eine unbeabsichtigte Verbrennung ausscheidet.

Um eine Beschädigung der im Schutzschlauch verlaufenden Kabel **13** und des Gasschlauches **11** zu vermeiden, ist im Bereich der Einführung in das Griffstück **1** ein Knickschutz **22** mit angeformter Zugentlastung vorgesehen, die durch eine Überwurfmutter **17** an dem Abschlußteil **16** befestigt ist. Um eine sichere Dichtheit des Gerätes zu gewährleisten, ist zwischen Abschlußteil **16** und Verbindungselement **14** ein Dichtring **15** vorgesehen.

Über eine Verbindungsleitung **20** ist der Kontakt zur Gegenpolplatte **2** hergestellt.

Eine Sterilisierung im Naßdampf ist möglich.

| 1 | - Griffstück |
| 2 | - Gegenpolplatte |
| 3 | - Düse |
| 4 | - Elektrodenhalter |
| 5 | - Gewinde |
| 6 | - Elektrode |
| 7 | - Gasaustritt |
| 8 | - Dichtring |
| 9 | - Zentrierelement |
| 10 | - Schlauchaufnahme |
| 11 | - Gasschlauch |
| 12 | - Leiterplatte |
| 13 | - Kabel |
| 14 | - Verbindungselement |
| 15 | - Dichtung |
| 16 | - Abschlußteil |
| 17 | - Überwurfmutter |
| 18 | - Schutzschlauch |
| 19 | - Versorgungseinheit |
| 20 | - Zuleitung |
| 21 | - Silikon |
| 22 | - Knickschutz |
| 23 | - Taster |
| 24 | - Messer |
| 25 | - Schieber |
| 26 | - Kontaktfeder |
| 27 | - Kontaktstift |
| 28 | - Führung |

**Patentansprüche**

1.  Mikro-Elektro-Schutzgas-Schweißgerät mit in einem Griffteil **1** untergebrachten Versorgungsleitungen für das Schutzgas und die hochfrequente Spannung **11**, **13** sowie einem Elektrodenhalter **4** und einer die Elektrode **6** umgebende Düse **3**, durch die das in einem auch die elektrischen Leiter **13** aufnehmenden Schlauch **11** von der Gasversorgungseinrichtung **19** zugeführte Gas auf das zu behandelnde Objekt austritt, **dadurch gekennzeichnet**, **daß** der Elektrodenhalter **4** und der Gaszuführ- und Verteilungskörper einstückig ausgeführt sind und der Elektrodenhalter **4** elektrisch leitend mit dem Zentrierelement **9** verbunden ist.

2.  Mikro-Eiektro-Schutzgas-Schweißgerät nach Anspruch 1, **dadurch gekennzeichnet**, **daß** die Eiektrode **6** aus einem nicht abnutzenden Material, vorzugsweise Wolfram besteht.

3.  Mikro-Elektro-Schutzgas-Schweißgerät nach Anspruch 1, **dadurch gekennzeichnet**, **daß** der Elektrodenhalter **4** in dem Zentrierelement **9** auswechselbar befestigt ist.

4. Mikro-Elektro-Schutzgas-Schweißgerät nach einem oder mehreren der vorhergehenden Ansprüche, **dadurch gekennzeichnet**, **daß** auf die rückwärtige Verlängerung des Elektrodenhalters **4** der Gaszuführungsschlauch **11** aufgesteckt ist.

5. Mikro-Elektro-Schutzgas-Schweißgerät nach Anspruch 1, **dadurch gekennzeichnet**, **daß** die hochfrequente Spannung über eine Leiterbahnen und Mikroschalter bzw. -taster aufnehmende Leiterplatte **12** dem Zentrierelement **9** zugeführt wird.

6. Mikro-Elektro-Schutzgas-Schweißgerät nach Anspruch 5, **dadurch gekennzeichnet**, **daß** die Mikroschalter und -taster über entsprechende in dem Schutzschlauch **18** zusammen mit dem Gasschlauch **11** gelagerte Kabel **13** mit der Gaszufuhr und Stromstärke steuernden Regeleinrichtung **19** verbunden sind.

7. Mikro-Elektro-Schutzgas-Schweißgerät nach Anspruch 1, **dadurch gekennzeichnet**, **daß** die Gasaustrittsdüse **3** aus einem elektrisch nicht leitenden, aber wärmeresistenten Material vorzugsweise Keramik besteht.

8. Mikro-Elektro-Schutzgas-Schweißgerät nach Anspruch 5, **dadurch gekennzeichnet**, **daß** der Zwischenraum zwischen den Kabeln **13** und dem Gasschlauch **11** mit hitzebeständigem Material vorzugsweise Silikon ausgefüllt ist.

Fig.1

Elektro-Generator

Gasversorgungs-einrichtung

Fig. 2